# EUROPÄISCHE PATENTSCHRIFT

(11) **EP 1 219 584 B1**
(45) Veröffentlichungstag und Bekanntmachung des Hinweises auf die Patenterteilung: **29.11.2006**
(21) Anmeldenummer: 01125953.8
(22) Anmeldetag: 31.10.2001
(51) Int. Cl.: C07C 29/141, C07C 31/125

(54) **Verfahren zur Hydrierung von Hydroformylierungsgemischen**
Process for the hydrogenation of hydroformylation mixtures
Procédé pour l'hydrogénation de mélanges d'hydroformylation

(30) Priorität: 14.12.2000 DE 10062448
(43) Veröffentlichungstag der Anmeldung: 03.07.2002
(73) Patentinhaber: Oxeno Olefinchemie GmbH, 45772 Marl (DE)
(72) Erfinder: Kaizik, Alfred, Dr., 45772 Marl (DE); Scholz, Bernhard, Dr., 45768 Marl (DE); Büschken, Wilfried, Dr., 45721 Haltern (DE); Tötsch, Walter, Dr., 45770 Marl (DE); Gubisch, Dietmar, Dr., 45772 Marl (DE); Schuler, Joachim, Dr., 45772 Marl (DE); Knoop, Cord, 45721 Haltern (DE)

(56) Entgegenhaltungen:
- EP-A- 0 987 241
- GB-A- 784 359
- GB-A- 808 336
- GB-A- 2 142 010
- US-A- 2 760 994
- US-A- 2 771 493
- US-A- 2 809 220
- US-A- 4 401 834

## Beschreibung

Die Erfindung betrifft ein Verfahren zur Hydrierung von Hydroformylierungsgemischen, d. h. zur Herstellung von Alkoholen durch Hydrierung von Aldehyden in flüssiger Phase in Gegenwart von Wasser.

Alkohole können durch katalytische Hydrierung von Aldehyden, die beispielsweise durch Hydroformylierung von Olefinen erhalten worden sind, hergestellt werden. Große Mengen von Alkoholen werden als Lösungsmittel und als Zwischenprodukte für die Herstellung vieler organischer Verbindungen verwendet. Wichtige Folgeprodukte von Alkoholen sind Weichmacher und Detergentien.

Es ist bekannt, Aldehyde katalytisch mit Wasserstoff zu Alkoholen zu reduzieren. Dabei werden häufig Katalysatoren eingesetzt, die mindestens ein Metall aus den Gruppen 1b, 2b, 6b, 7b, und/oder 8 des Periodensystems der Elemente enthalten. Die Hydrierung von Aldehyden kann kontinuierlich oder diskontinuierlich mit pulverförmigen oder stückigen Katalysatoren in der Gas- oder Flüssigphase durchgeführt werden.

Für die technische Herstellung von Alkoholen durch Hydrierung von Aldehyden aus dem Oxo-Prozess (Hydroformylierung von Olefinen) werden, vor allem bei Großprodukten, kontinuierliche Verfahren mit im Festbett angeordneten Katalysatoren in der Gas- oder Flüssigphase bevorzugt.

Im Vergleich zur Gasphasenhydrierung weist die Flüssigphasenhydrierung die günstigere Energiebilanz und die höhere Raum-Zeit-Ausbeute auf. Mit steigender Molmasse des zu hydrierenden Aldehyds, d. h. mit steigenden Siedepunkten, nimmt der Vorteil der günstigeren Energiebilanz zu. Höhere Aldehyde mit mehr als 7 Kohlenstoffatomen werden demnach vorzugsweise in der Flüssigphase hydriert.

Die Hydrierung in der Flüssigphase hat allerdings den Nachteil, dass aufgrund der hohen Konzentrationen sowohl an Aldehyden als auch an Alkoholen die Bildung von Hochsiedern durch Folge- und Nebenreaktionen begünstigt wird. So können Aldehyde leichter Aldolreaktionen (Addition und/oder Kondensation) eingehen und mit Alkoholen Halb- oder Vollacetale bilden. Die entstandenen Acetale können unter Abspaltung von Wasser oder Alkohol Enolether bilden, die unter den Reaktionsbedingungen zu den gesättigten Ethern hydriert werden. Diese sekundären Nebenprodukte vermindern somit die Ausbeute. Die als Hochsieder bezeichneten Nebenprodukte können bestenfalls teilweise in nachgeschalteten Anlagen in Wertprodukte, wie Ausgangsaldehyde und Zielalkohole, zurückgespalten werden.

Technische Aldehydgemische, die zur Hydrierung eingesetzt werden, enthalten häufig bereits Hochsieder in unterschiedlicher Konzentration.

Bei der Hydroformylierung von Olefinen in Gegenwart von Kobalt-Katalysatoren werden Rohaldehyde erhalten, die neben den Ameisensäureestern (Formiaten) zusätzlich Aldolprodukte, höhere Ester und Ether sowie Acetale als Hochsieder enthalten. Werden diese Gemische in der Gasphase hydriert, so kann der größte Teil der Hochsieder im Verdampfer abgetrennt und in einem separaten Verfahrensschritt zu Wertprodukten aufgearbeitet werden.

Bei der Flüssigphasenhydrierung dagegen bleiben die Hochsieder im Reaktorzulauf. Sie werden in der Hydrierstufe zum größten Teil hydriert, sodass aus ihnen kein Wertprodukt mehr gewonnen werden kann.

In US 5 059 710 wird die Ausbeute an Alkoholen durch Hydrierung von Rohaldehyden dadurch erhöht, dass in einer der Hydrierung vorgeschalteten Verfahrenstufe ein Teil der Hochsieder bei erhöhter Temperatur mit Wasser zu Aldehyden oder Alkoholen zurückgespalten wird. Hydrolyse und Hydrierung sind daher getrennte Verfahrensstufen, wobei über den Wassergehalt des zu hydrierenden Gemischs keine Aussage gemacht wird.

Ein ähnliches Verfahren wird in US 4 401 834 offenbart. Auch hier erfolgt die Spaltung von Hochsiedern in Gegenwart von Wasser vor dem eigentlichen Hydrierungsschritt.

In GB 2 142 010 wird ein Verfahren zur Hydrierung von rohen Aldehyden mit 6 bis 20 C-Atomen, die Hochsieder und geringe Mengen an Schwefelverbindungen enthalten, zu den entsprechenden gesättigten Alkoholen beansprucht. Die Hydrierung erfolgt in zwei hintereinander geschalteten Reaktoren. Der erste Reaktor enthält einen MoS₂/C- und der zweite Reaktor einen Ni/Al₂O₃-Katalysator. Die Hydrierung wird in beiden Reaktoren unter Zusatz von bis zu 10 % Wasserdampf, bezogen auf den Eduktstrom, im Temperaturbereich 180 bis 260 °C und einem Wasserstoffpartialdruck von 150 bis 210 bar mit großem Wasserstoffüberschuss durchgeführt. Dieser ist nach den Beispielen so groß, dass sich das zugesetzte Wasser praktisch nur in der Gasphase befindet. Das Ziel dieses Verfahrens liegt in der Zurückdrängung der Bildung von Kohlenwasserstoffen durch Hydrogenolyse der Alkohole. Über eine Zunahme bzw. Abnahme von Hochsiedern und Formiaten bei der Hydrierung werden keine Aussagen gemacht.

In GB 808,336 wird die Hydrierung eines C₈-Aldehydgemisches an einem Molybdänkatalysator beschrieben, wobei das Hydriergemisch 5 bis 6 % Wasser aufweist.

In US 2 809 220 wird eine Flüssigphasenhydrierung von Hydroformylierungsgemischen in Gegenwart von Wasser beschrieben. Als Katalysator werden schwefelhaltige Katalysatoren eingesetzt. Die Hydrierung wird im Druckbereich von 105 bis 315 bar und im Temperaturbereich von 204 bis 315 °C in Gegenwart von 1 bis 10 % Wasser, bezogen auf Edukt, durchgeführt. Um das zugesetzte Wasser in der Gasphase zu halten, wird ein großer Überschuss an Wasserstoff (892 bis 3566 Nm³ Wasserstoff je m³ Edukt) eingesetzt. Bezüglich des hohen Wasserstoffüberschusses wird auf die Diskussion von GB 2 142 010 verwiesen. Nachteilig an diesem Verfahren ist weiterhin der hohe spezifische Energieverbrauch.

In GB 784,359 und US 2,760,994, beide Gulf Research & Development Company, wird ein Verfahren zur Hydrierung von Hydroformylierungsgemischen beschrieben, bei dem in die Hydrierungszone ein Gemisch eingespeist wird, welches ein Volumenverhältnis von Wasser zu Hydroformylierungsgemisch von 1,15 zu 1 bis 2 zu 1 aufweist. Die Hydrierung wird bei einem hohen Wasserstoffüberschuss durchgeführt.

In US 2,771,493 wird ein Verfahren zur Hydrierung von Aldehyden beschrieben, wobei zur Kühlung bzw. zur Einstellung der Reaktionstemperatur große Mengen Wasserstoff eingesetzt werden. Bei der Verwendung von Molybdänsulfid-Katalysatoren wird in US 2,771,493 die Zugabe von Wasser zum zu hydrierenden Gemisch vorgeschlagen.

Ein weiteres Verfahren zur Hydrierung von Hydroformylierungsgemisches offenbart DE 198 42 370. Hier wird beschrieben, wie Hydroformylierungsgemische in der Flüssigphase an Kupfer, Nickel und Chrom enthaltenden Trägerkatalysatoren hydriert werden können. Je nach Herstellverfahren der Hydroformylierungsgemische (Rhodium- oder Kobaltverfahren) enthalten diese Gemische Wasser. Das offenbarte Verfahren ist für die selektive Hydrierung der Aldehyde zu Alkoholen, ohne Hydrierung der in der Hydroformylierung nicht umgesetzten Olefine ausgelegt, d. h. die Hochsieder (vor allem Acetale) werden nicht zu Wertprodukt umgesetzt. Dies ist wirtschaftlich ungünstig und daher verbesserungsfähig.

Da die bekannten Verfahren hinsichtlich der Wirtschaftlichkeit (geringes Investment, hohe Produktausbeute und geringer Energieaufwand) nicht optimal sind, bestand die Aufgabe, ein neues Verfahren zur Hydrierung von Aldehyden oder Aldehydgemischen zu den entsprechenden gesättigten Alkoholen zu entwickeln, das die Vorteile der Gasphasenhydrierung (hohe Selektivität) mit der der Flüssigphasenhydrierung (geringer Energieaufwand, hohe Raum-Zeit-Ausbeute) vereint.

Es wurde gefunden, dass die Ausbeute an Alkoholen bei der Flüssigphasenhydrierung von Aldehyden oder technischen Aldehydgemischen erhöht wird, wenn die Hydrierung in Gegenwart von Wasser durchgeführt wird, wobei das Wasser unter den Reaktionsbedingungen hauptsächlich in der flüssigen Phase vorhanden ist und keine separate flüssige Wasserphase entsteht.

Der Gegenstand der Erfindung ist demnach ein Verfahren zur kontinuierlichen Hydrierung von Reaktionsgemischen aus der Hydroformylierung von Olefinen mit 4 bis 16 Kohlenstoffatomen in der homogenen Flüssigphase an Festbettkatalysatoren, die mindestens ein Element der achten Nebengruppe des Periodensystems der Elemente enthalten, wobei die homogene Flüssigphase des Reaktoraustrags noch zwischen 0,05 und 10 Gew.-% Wasser enthält und im stationärem Zustand des Verfahrens 3 bis 50 % mehr Wasserstoff eingespeist wird, als durch die Hydrierung verbraucht wird.

Das erfindungsgemäße Verfahren weist eine Reihe von Vorteilen auf. Bei der Hydrierung von reinen Aldehyden in der flüssigen Phase in Gegenwart von homogen gelöstem Wasser entsprechen die Ausbeuten und Selektivitäten denen von Gasphasenhydrierungen, jedoch ist der Energieaufwand beträchtlich geringer.

Werden Aldehyde oder Aldehydgemische, die Formiate und Hochsieder enthalten, wobei letztere überwiegend aus Aldolprodukten und Acetalen bestehen, in der flüssigen Phase in Gegenwart von Wasser hydriert, werden Formiate (Ameisensäureester) praktisch vollständig und Hochsieder zum Teil in Alkohole umgewandelt. Dadurch entsteht eine größere Alkoholmenge als der Aldehydmenge im Einsatzgemisch äquivalent ist.

Bei der Hydrierung von reinen Aldehyden, bzw. Hochsieder-armen Aldehyden nach dem erfindungsgemäßen Verfahren wird die Hochsieder-Bildung bei der Hydrierung deutlich herabgesetzt und dadurch die Selektivität der Hydrierung entscheidend verbessert. Um die Selektivitäts- und Ausbeute-steigernde Wirkung des Wassers zu erhalten, ist es notwendig, dass das Wasser in der Flüssigphase vorliegt. Ein Wassergehalt in der Gasphase ist daher nicht entscheidend.

Die Edukte für die Herstellung der Aldehyde bzw. des Reaktionsgemisches durch Hydroformylierung sind Olefine oder Gemische von Olefinen mit 4 bis 16, bevorzugt mit 6 bis 12 Kohlenstoffatomen, mit end- oder innenständigen C-C-Doppelbindungen, wie z. B. 1-Buten, 2-Buten, Isobuten, 1- oder 2-Penten, 2-Methylbuten-1, 2-Methylbuten-2, 3-Methylbuten-1, 1-,2- oder 3-Hexen, das bei der Dimerisierung von Propen anfallende C₆-Olefingemisch (Dipropen), Heptene, 2-oder 3-Methyl-1-hexen, Octene, 2-Methylheptene, 3-Methylheptene, 5-Methylhepten-2, 6-Methylhepten-2, 2-Ethylhexen-1, das bei der Dimerisierung von Butenen anfallende Gemisch der isomeren C₈-Olefine (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Pentadecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge. Ebenfalls können Olefine oder Olefingemische, die durch Fischer-Tropsch-Synthese erzeugt worden sind, sowie Olefine, die durch Oligomerisierung von Ethen erhalten wurden oder Olefine, die über Methathesereaktionen zugänglich sind, eingesetzt werden. Bevorzugte Edukte für die Herstellung der Hydroformylierungsgemische sind C₈-, C₉-, C₁₂-, C₁₅- oder C₁₆-Olefingemische.

Die Olefine werden in üblicher Weise hydroformyliert und ergeben dann die Edukte für das erfindungsgemäße Hydrierverfahren. Man arbeitet in der Regel mit Rhodium- oder Kobaltkatalysatoren sowie mit oder ohne komplexstabilisierende Zusätze, wie organischen Phosphinen oder Phosphiten. Die Temperaturen und Drücke können, je nach Katalysator oder Olefin, in weiten Grenzen variieren. Eine Beschreibung der Hydroformylierung von Olefinen findet sich z. B. bei J. Falbe, New Syntheses with Carbon Monoxide, Springer-Verlag, Heidelberg-New York, 1980, Seite 99 ff., sowie bei Kirk-Othmer, Encyclopedia of Chemical Technology, Band 17, 4. Auflage, John Wiley & Sons, Seiten 902 bis 919 (1996).

Bevorzugt werden im Verfahren der Erfindung Hydroformylierungsgemische, hergestellt aus C₈-, C₁₂-Olefinen oder C₈-, C₁₂-Olefingemischen eingesetzt.

Die Aldehydmengen im Reaktorzulauf können auf Konzentrationen von 1-35 Gew.-%, bevorzugt 5-20 Gew.-% beschränkt werden.

Die Reaktionsgemische der Hydroformylierung werden zweckmäßig zunächst vom Katalysator befreit. Wenn ein Kobaltkatalysator verwendet worden ist, kann dies durch Druckentlastung, Oxidation der im Hydroformylierungsgemisch verbliebenen Kobaltcarbonylverbindungen in Gegenwart von Wasser oder wässriger Säure und Abtrennung der wässrigen Phase geschehen. Entkobaltungsverfahren sind gut bekannt, siehe z. B. J. Falbe, a. a. O., Kirk-Othmer, a. a. O., 164, 175, BASF-Verfahren.

Wenn eine Rhodiumverbindung als Hydroformylierungskatalysator eingesetzt wird, kann man sie z. B mittels Dünnschichtverdampfung als Destillationsrückstand abtrennen.

Die vom Hydroformylierungskatalysator befreiten Reaktionsgemische der Kobalt-katalysierten Hydroformylierung enthalten im Allgemeinen 3 bis 40 Massen-%, meistens 5 bis 30 Massen-% Leichtsieder, hauptsächlich nicht umgesetzte Olefine, daneben die entsprechenden gesättigten Kohlenwasserstoffe sowie 0,05 bis 5 Massen-% Wasser, 30 bis 90 Massen-% Aldehyde, 5 bis 60 Massen-% Alkohole, bis zu 10 Massen-% Formiate dieser Alkohole und 3 bis 15 Massen-% Hochsieder.

Im Unterschied zur Kobalt-katalysierten Hydroformylierung enthalten die Reaktionsgemische aus der Rhodium-katalysierten Hydroformylierung praktisch kein Wasser. Hier muss Wasser entsprechend zudosiert werden.

Es sei jedoch betont, dass das erfindungsgemäße Verfahren auch mit Hydroformylierungsgemischen ausgeführt werden kann, deren Zusammensetzung in dieser und bzw. oder jener Beziehung nicht diesen Angaben entspricht. So können beispielsweise vor der Hydrierung die Kohlenwasserstoffe (Olefine und Paraffine) vom Hydroformylierungsgemisch abgetrennt werden.

Zur Hydrierung von Aldehyden mittels des erfindungsgemäßen Verfahrens in flüssiger Phase in Gegenwart von Wasser können stückige, im Festbett angeordnete Katalysatoren verwendet werden. Diese können ein oder mehrere Metalle der Gruppen 1 b, 2b, 6b, 7b und/oder 8 des Periodensystems, insbesondere Nickel, Kupfer und Chrom, enthalten. Es können Katalysatoren auf oxidischen Trägern, wie Aluminium-, Silizium-, Titanoxid, Alumosilikate oder trägerfreie Katalysatoren zum Einsatz kommen. Trägerfreie Katalysatoren enthalten im Allgemeinen ca. 0.2 bis 30 Gew.-% Nickel, 0.3 bis 40 Massen-% Kupfer und 18 bis 40 Massen.-% Chrom. Die Katalysatoren können weiterhin bis zu 20 Massen -% an basischen Stoffen, wie Alkali- oder Erdalkalioxiden oder -hydroxiden, sowie andere, inerte oder eigenschaftsmodifizierende Stoffe in derselben Mengen enthalten, beispielsweise Graphit. Die erfindungsgemäß eingesetzten Katalysatoren enthalten keinen Schwefel oder Schwefelverbindungen.

Bevorzugte Katalysatoren im erfindungsgemäßen Verfahren zur Hydrierung von Aldehyden zu Alkoholen sind Trägerkatalysatoren. Die Katalysatoren enthalten jeweils 0,3 bis 15 Gew.-% Kupfer und Nickel sowie als Aktivatoren 0,05 bis 3,5 Gew.-% Chrom und vorteilhaft 0 bis 1,6 Gew.-% eines Alkalimetalls. Das Trägermaterial besteht vorzugsweise aus Aluminiumoxid und/oder Siliciumoxid.

Die Katalysatoren werden vorteilhaft in einer Form eingesetzt, in der sie einen geringen Strömungswiderstand bieten, z. B. in Form von Granalien, Pellets oder Formkörpern, wie Tabletten, Zylinder, Kugeln, Strangextrudaten oder Ringen. Sie werden zweckmäßig vor ihrem Einsatz durch Erhitzen im Wasserstoffstrom, beispielsweise auf 140 bis 250 °C, aktiviert, sofern sie nicht im Hydrierreaktor reduziert werden. Beispielsweise wird ein Verfahren zur Reduktion mit Wasserstoff in Gegenwart einer flüssigen Phase in DE 199 33 348.3 beschrieben.

Erfindungsgemäß wird die Hydrierung in der homogenen flüssigen Phase in Gegenwart von Wasser durchgeführt, wobei die homogene Flüssigphase des Reaktoraustrags zwischen von 0,05 bis 10 Gew.-%, bevorzugt 0,5 bis 8 Gew.-%, besonders bevorzugt 1 bis 5 Gew.-% Wasser enthält. Die genannten Wassergehalte sind unabhängig vom Wasserverbrauch durch chemische Reaktionen und vom Wasseraustrag mit dem Hydrierabgas zu verstehen. Unter den Reaktionsbedingungen der Hydrierung befindet sich das Wasser hauptsächlich in der organischen Edukt-Produktphase und nur zum geringen Teil in der Gasphase. Im Idealfall enthält die Gasphase kein Wasser. Eine weitere, flüssige Wasserphase ist nicht vorhanden. Die spezifische Wassermenge in der organischen Phase ist durch die Löslichkeit des Wassers, den Dampfdruck des Wassers und das Phasenverhältnis (Gas zu Flüssigkeit) unter den Reaktionsbedingungen bedingt. Die mindest notwendige Wassermenge ist diejenige, die für die Hydrolyse von Ameisensäureestern, Vollacetalen, Enolethern, Aldolkondensationprodukten und gegebenenfalls anderen hydrolysierbaren Stoffen verbraucht wird. Enthält das Edukt große Anteile von hydrolysierbaren Verbindungen, kann es notwendig sein, um die Bildung einer zweiten wässrigen Phase im Hydrierreaktor zu verhindern, nur einen Teil des erforderlichen Wassers zu Beginn zuzugeben. Der andere Teil wird während der Hydrierung je nach Wasserverbrauch eingespeist. Dies geschieht bei Verwendung nur eines Reaktors an einer oder einigen Stellen des Reaktors, bei Verwendung mehrerer hintereinandergeschalteter Reaktoren zweckmäßig vor den einzelnen Reaktoren. Damit sich kein Aldehyd, geschützt als Halb- oder Vollacetal, der Hydrierung entziehen kann, kann der Hydrieraustrag (bei mehreren Reaktoren der letzte Reaktor) noch Wasser enthalten. Der Wassergehalt in der homogenen Flüssigphase des Reaktoraustrags kann 0,05 bis 10 Massen-%, vorzugsweise 0,5 bis 8 Massen-% betragen.

Für das erfindungsgemäße Verfahren können unterschiedliche Verfahrensvarianten gewählt werden. Es kann adiabatisch, oder praktisch isotherm, d. h. mit einem Temperaturanstieg kleiner 10°C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die Aldehyde oder Aldehydgemische in Gegenwart von Wasser im geraden Durchgang oder mit Produktrückführung zu hydrieren.

Das erfindungsgemäße Verfahren wird in der Rieselphase oder bevorzugt in der Flüssigphase in Dreiphasenreaktoren im Gleichstrom durchgeführt, wobei der Wasserstoff in an sich bekannter Weise in dem flüssigen Aldehyd-Strom fein verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120 , insbesondere von 25 bis 80 m³ pro m² Querschnitt des leeren Reaktors und Stunde betrieben. Wird ein Reaktor isotherm und im geraden Durchgang betrieben, so kann die spezifische Katalysatorbelastung (LHSV) Werte zwischen 0,1 und 10 h⁻¹ annehmen.

Bei der Hydrierung von Hydroformylierungsgemischen mit 8 bis 17 C-Atomen, wie beispielsweise Isononanal oder Tridecanal, werden bevorzugt mehrere hintereinandergeschaltete Reaktoren verwendet. Dabei wird (werden) der erste in Schlaufenfahrweise und der (die) folgende(n) Reaktor(en) in Schlaufenfahrweise oder im geraden Durchgang betrieben. Als Reaktor, der in Schlaufenfahrweise betrieben wird, kann beispielsweise ein Schachtofen mit einem Wärmeaustauscher im Außenkreislauf oder ein Rohrbündelreaktor verwendet werden.

Zur Minimierung von Nebenreaktionen und somit Erhöhung der Alkoholausbeute ist es zweckmäßig, die Aldehydkonzentration im Reaktorzulauf zu begrenzen. Insbesondere bei der Hydrierung von Hydroformylierungsgemische mit 8 bis 17 C-Atomen liegt der Aldehydgehalt im Reaktorzulauf zwischen 1 und 35 %, bevorzugt zwischen 5 und 25 %. Der gewünschte Konzentrationsbereich kann bei Reaktoren, die in Schlaufenfahrweise betrieben werden, durch die Zirkulationsrate (Mengenverhältnis von rückgeführten Hydrieraustrag zu Edukt) eingestellt werden.

Das erfindungsgemäße Verfahren wird in einem Druckbereich von 5 bis 100 bar, insbesondere zwischen 5 und 40 bar, ganz besonders im Bereich von 10 bis 25 bar durchgeführt. Die Hydriertemperaturen liegen zwischen 120 und 220°C, insbesondere zwischen 140 und 190 °C.

Der zur Hydrierung erforderliche Wasserstoff wird möglichst rein mit nur geringem Überschuss eingesetzt, sodass nur wenig Wasser in die Gasphase übergeht und mit ihr ausgetragen wird. Die eingesetzte Wasserstoffmenge beträgt bei jedem Reaktor 103 bis 150 % der durch Reaktion verbrauchten Menge, insbesondere 103 bis 120 %. Anders ausgedrückt, wird der bei der Hydrierung verbrauchte Wasserstoff in einem 3- bis 50%igen, bevorzugt in einem 3- bis 20%igen, besonders bevorzugt in einem 5- bis 10%igen Überschuss ersetzt.

Das Hydrierprodukt wird destillativ aufgearbeitet. Dies geschieht bei Normaldruck oder verminderten Druck. Bei hochsiedenden Alkoholen wird die Destillation bei vermindertem Druck bevorzugt.

Die folgenden Beispiele sollen die Erfindung weiter erläutern, nicht aber ihren Anwendungsbereich beschränken, der sich aus den Patentansprüchen ergibt.

### Beispiel 1 (Verqleichsbeispiel):

C₉-Aldehydhydrierung in der Flüssigphase / Roh-Aldehyd - Wasser-freies Edukt

Ein Liter eines Reaktionsaustrags der Co-katalysierten Hydroformylierung von Dibuten mit 1,15 Gew.-% Wasser und 5,16 Gew.-% Hochsieder wurde nach Entfernung des Wassers (bis auf 110 ppm Restwasser) durch eine Labordestillation in einer Kreislaufapparatur bei 180 °C und 25 bar absolut an 100 g eines Cu/Cr/Ni-Katalysators auf Al₂O₃-Träger in der Flüssigphase hydriert. Die Abgasmenge betrug 1 Nl/h. Die Edukt- und Produkt-Analysen sind in Tabelle 1 wiedergegeben.

**Tabelle 1:**

| Versuchszeit | C₈-KWST | C₉-al | Formiat | C₉-ol | Hochsieder |
|---|---|---|---|---|---|
| (Stunden) | (Gew-%) | (Gew-%) | (Gew-%) | (Gew-%) | (Gew-%) |
| 0 | 9,32 | 46,50 | 3,24 | 35,79 | 5,16 |
| 1 | 9,28 | 0,29 | 1,35 | 83,64 | 5,44 |
| 2 | 9,25 | 0,18 | 0,45 | 84,03 | 6,10 |
| 3 | 9,18 | 0,15 | 0,22 | 84,00 | 6,45 |

Wie man aus der Tabelle 1 entnehmen kann, werden die Hochsieder bei der Hydrierung von Isononanal bei Abwesenheit von Wasser im Edukt nicht gespalten, sondern gebildet.

### Beispiel 2 (gemäß der Erfindung):

### C₉-Aldehydhydrierung /Roh-Aldehyd - Wasser-haltiges Edukt

Ein Liter eines Reaktionsaustrags der Co-katalysierten Hydroformylierung von Dibuten mit 1,5 Gew.-% Restwassers und 5,35 Gew.-% Hochsieder wurde in einer Kreislaufapparatur bei 180 °C und 25 bar absolut an 100g eines Cu/Cr/Ni-Katalysators auf Al₂O₃-Träger in der Flüssigphase hydriert. Die Abgasmenge betrug 1 Nl/h. Die Edukt- und Produkt-Analysen sind, wasserfrei gerechnet, in Tabelle 2 wiedergegeben.

**Tabelle 2:**

| Versuchszeit | C₈-KWST | C₉-al | Formiat | C₉-ol | Hochsieder |
|---|---|---|---|---|---|
| (Stunden) | (Gew-%) | (Gew-%) | (Gew-%) | (Gew-%) | (Gew-%) |
| 0 | 9,12 | 47,20 | 3,16 | 37,17 | 5,35 |
| 1 | 9,18 | 0,34 | 0,32 | 85,72 | 4,45 |
| 2 | 9,15 | 0,20 | <0,01 | 86,67 | 3,84 |
| 3 | 9,09 | 0,18 | <0,01 | 86,86 | 3,73 |

Wie man aus der Tabelle 2 entnehmen kann, werden bei der Hydrierung von Roh-Isononanal in Gegenwart von Wasser im Edukt die Hochsieder teilweise zu Wertprodukten gespalten und die Formiate schneller und praktisch quantitativ abgebaut. Nach der Hydrierung enthielt der flüssige Reaktoraustrag 1,01 Gew.-% Wasser.

### Beispiel 3 (gemäß der Erfindung):

### C₉-Aldehydhydrierung / Hochsieder-armes und Wasser-haltiges Edukt

Ein Liter eines Reaktionsaustrags der Co-katalysierten Hydroformylierung von Dibuten mit 1,20 Gew.-% Wasser wurde nach weitgehendener Entfernung der Hochsieder (von 4,65 Gew.-% auf 0,13 Gew.-%) durch eine Labordestillation in einer Kreislaufapparatur bei 180 °C und 25 bar absolut an 100 g eines Cu/Cr/Ni-Katalysators auf Al₂O₃-Träger in der Flüssigphase hydriert. Die Abgasmenge betrug 1 Nl/h. Die Edukt- und Produkt-Analysen sind in Tabelle 3 wiedergegeben.

**Tabelle 3:**

| Versuchszeit | C₈-KWST | C₉-al | Formiat | C₉-ol | Hochsieder |
|---|---|---|---|---|---|
| (Stunden) | (Gew-%) | (Gew-%) | (Gew-%) | (Gew-%) | (Gew-%) |
| 0 | 7,40 | 52,86 | 3,44 | 36,17 | 0,13 |
| 1 | 7,27 | 0,26 | 0,18 | 90,83 | 1,46 |
| 2 | 7,29 | 0,21 | 0,01 | 90,87 | 1,48 |
| 3 | 7,32 | 0,19 | <0,01 | 90,86 | 1,49 |

Wie man aus der Tabelle 3 entnehmen kann, werden die Isononylformiate bei der Hydrierung von Isononanal in Gegenwart von im Edukt homogen gelöstem Wasser sehr schnell zum Wertprodukt Isononanol abgebaut.
Die Hochsiedergehalte nehmen nach einer Versuchszeit von einer Stunde einen konstanten Wert von rd.1,46 Gew.-% an.
Nach der Hydrierung enthielt der Reaktoraustrag 0,70 Gew.-% Wasser.

### Beispiel 4 (Vergleich):

### C₉-Aldehydhydrierung / Hochsieder-armes und Wasser-freies Edukt

Ein Liter eines Reaktionsaustrags der Co-katalysierten Hydroformylierung von Dibuten wurde nach Entfernung des Restwassers (von 1 Gew.-% auf 150 ppm) und Hochsieder durch eine Labordestillation in einer Kreislaufapparatur bei 180 °C und 25 bar absolut an 100g eines Cu/Cr/Ni-Katalysators auf Al₂O₃-Träger in der Flüssigphase hydriert. Die Abgasmenge betrug 1 Nl/h. Die Edukt- und Produkt-Analysen sind in Tabelle 4 wiedergegeben.

**Tabelle 4:**

| Versuchszeit | C₈-KWST | C₉-al | Formiat | C₉-ol | Hochsieder |
|---|---|---|---|---|---|
| (Stunden) | (Gew-%) | (Gew-%) | (Gew-%) | (Gew-%) | (Gew-%) |
| 0 | 6,95 | 51,50 | 3,64 | 37,79 | 0,13 |
| 1 | 6,97 | 0,33 | 1,21 | 87,05 | 4,44 |
| 2 | 6,98 | 0,19 | 0,50 | 89,21 | 3,13 |
| 3 | 6,94 | 0,15 | 0,27 | 89,63 | 3,01 |

Wie man aus der Tabelle 4 entnehmen kann, werden im Vergleich zum Beispiel 3 die Isononylformiate bei der Hydrierung von Isononanal in Abwesenheit von Wasser im Edukt nur langsam zum Wertprodukt Isononanol abgebaut. Darüber hinaus werden in Abwesenheit von Wasser deutlich mehr Hochsieder gebildet.

## Patentansprüche

1. Verfahren zur kontinuierlichen Hydrierung von Reaktionsgemischen aus der Hydroformylierung von Olefinen mit 4 bis 16 Kohlenstoffatomen in der homogenen Flüssigphase an Festbettkatalysatoren, die mindestens ein Element der achten Nebengruppe des Periodensystems der Elemente enthalten,
**dadurch gekennzeichnet,**
**dass** die homogene Flüssigphase des Reaktoraustrags noch zwischen 0,05 und 10 Gew.-% Wasser enthält
und
im stationärem Zustand des Verfahrens 3 bis 50 % mehr Wasserstoff eingespeist wird, als durch die Hydrierung verbraucht wird.

2. Verfahren nach Anspruch 1,
**dadurch gekennzeichnet,**
**dass** die homogene Flüssigphase des Reaktoraustrags zwischen 0,5 und 8 Gew.-% Wasser enthält.

3. Verfahren nach Anspruch 1 und 2,
**dadurch gekennzeichnet,**
**dass** die homogene Flüssigphase des Reaktoraustrags zwischen 1 und 5 Gew.-% Wasser enthält.

4. Verfahren nach einem der Ansprüche 1 bis 3,
**dadurch gekennzeichnet,**
**dass** die Hydrierung bei einem Druck von 5 bis 100 bar, insbesondere bei 5 bis 40 bar durchgeführt wird.

5. Verfahren nach einem der Ansprüche 1 bis 4,
**dadurch gekennzeichnet,**
**dass** die Hydrierung bei einer Temperatur von 120 bis 220 °C durchgeführt wird.

6. Verfahren nach einem der Ansprüche 1 bis 5,
**dadurch gekennzeichnet;**
**dass** Katalysatoren eingesetzt werden, die zwei Metalle aus der Gruppe Kupfer, Chrom und Nickel enthalten.

7. Verfahren nach einem der Ansprüche 1 bis 5,
**dadurch gekennzeichnet,**
**dass** Katalysatoren eingesetzt werden, die Kupfer, Chrom und Nickel enthalten.

8. Verfahren nach Anspruch 7,
**dadurch gekennzeichnet,**
**dass** der Festbettkatalysator je 0,3 bis 15 Gew.-% Kupfer und Nickel, 0,05 bis 3,5 Gew.-% Chrom und 0 bis 1,6 Gew.-% eines Alkalimetalls enthält.

9. Verfahren nach einem der Ansprüche 1 bis 8,
**dadurch gekennzeichnet,**
**dass** der Festbettkatalysator Siliciumdioxid und/oder Aluminiumoxid als Trägermaterial enthält.

10. Verfahren nach einem der Ansprüche 1 bis 9,
**dadurch gekennzeichnet,**
**dass** die Aldehydkonzentration im Reaktorzulauf 1 bis 35 % beträgt.

11. Verfahren nach einem der Ansprüche 1 bis 10,
**dadurch gekennzeichnet,**
**dass** die Aldehydkonzentration im Reaktorzulauf 5 bis 20 % beträgt.

12. Verfahren nach einem der Ansprüche 1 bis 11,
**dadurch gekennzeichnet,**
**dass** Hydroformylierungsgemische, hergestellt auf C₈-Olefinen oder C₈-Olefingemischen, eingesetzt werden.

13. Verfahren nach einem der Ansprüche 1 bis 11,
**dadurch gekennzeichnet,**
**dass** Hydroformylierungsgemische, hergestellt aus C₁₂-Olefinen oder C₁₂-Olefingemischen, eingesetzt werden.

## Claims

1. Process for the continuous hydrogenation of reaction mixtures from the hydroformylation of olefins having from 4 to 16 carbon atoms in the homogeneous liquid phase over fixed-bed catalysts comprising at least one element of transition group eight of the Periodic Table of the Elements,
**characterized in that**
the homogeneous liquid phase of the output from the reactor still contains from 0.05 to 10% by weight of water
and,
in steady-state operation of the process, from 3 to 50% more hydrogen is fed in than is consumed by the hydrogenation.

2. Process according to Claim 1, **characterized in that** the homogeneous liquid phase of the output from the reactor contains from 0.5 to 8% by weight of water.

3. Process according to Claim 1 or 2, **characterized in that** the homogeneous liquid phase of the output from the reactor contains from 1 to 5% by weight of water.

4. Process according to any of Claims 1 to 3, **characterized in that** the hydrogenation is carried out at a pressure of from 5 to 100 bar, in particular from 5 to 40 bar.

5. Process according to any of Claims 1 to 4, **characterized in that** the hydrogenation is carried out at a temperature of from 120 to 220°C.

6. Process according to any of Claims 1 to 5, **characterized in that** catalysts comprising two metals selected from the group consisting of copper, chromium and nickel are used.

7. Process according to any of Claims 1 to 5, **characterized in that** catalysts comprising copper, chromium and nickel are used.

8. Process according to Claim 7, **characterized in that** the fixed-bed catalyst comprises from 0.3 to 15% by weight of copper, from 0.3 to 15% by weight of nickel, from 0.05 to 3.5% by weight of chromium and from 0 to 1.6% by weight of an alkali metal.

9. Process according to any of Claims 1 to 8, **characterized in that** the fixed-bed catalyst comprises silicon dioxide and/or aluminum oxide as support material.

10. Process according to any of Claims 1 to 9, **characterized in that** the aldehyde concentration in the feed to the reactor is from 1 to 35%.

11. Process according to any of Claims 1 to 10, **characterized in that** the aldehyde concentration in the feed to the reactor is from 5 to 20%.

12. Process according to any of Claims 1 to 11, **characterized in that** hydroformylation mixtures prepared from C₈-olefins or C₈-olefin mixtures are used.

13. Process according to any of Claims 1 to 11, **characterized in that** hydroformylation mixtures prepared from C₁₂-olefins or C₁₂-olefin mixtures are used.

## Revendications

1. Procédé pour l'hydrogénation continue de mélanges réactionnels provenant de l'hydroformylation d'oléfines comprenant 4 à 16 atomes de carbone dans la phase liquide homogène sur des catalyseurs en lit fixe qui contiennent au moins un élément du huitième groupe secondaire du système périodique des éléments, **caractérisé en ce que** la phase liquide homogène de l'évacuation du réaction contient encore entre 0,05 et 10% en poids d'eau et on introduit à l'état stationnaire du procédé 3 à 50% d'hydrogène en plus de l'hydrogène consommé par l'hydrogénation.

2. Procédé selon la revendication 1, **caractérisé en ce que** la phase liquide homogène de l'évacuation du réacteur contient entre 0,5 et 8% en poids d'eau.

3. Procédé selon la revendication 1 et 2, **caractérisé en ce que** la phase liquide homogène de l'évacuation du réacteur contient entre 1 et 5% en poids d'eau.

4. Procédé selon l'une quelconque des revendications 1 à 3, **caractérisé en ce que** l'hydrogénation est réalisée à une pression de 5 à 100 bars, en particulier de 5 à 40 bars.

5. Procédé selon l'une quelconque des revendications 1 à 4, **caractérisé en ce que** l'hydrogénation est réalisée à une température de 120 à 220°C.

6. Procédé selon l'une quelconque des revendications 1 à 5, **caractérisé en ce qu'**on utilise des catalyseurs qui contiennent deux métaux du groupe formé par le cuivre, le chrome et le nickel.

7. Procédé selon l'une quelconque des revendications 1 à 5, **caractérisé en ce qu'**on utilise des catalyseurs qui contiennent du cuivre, du chrome et du nickel.

8. Procédé selon la revendication 7, **caractérisé en ce que** le catalyseur en lit fixe contient à chaque fois 0,3 à 15% en poids de cuivre et de nickel, 0,05 à 3,5% en poids de chrome et 0 à 1,6% en poids d'un métal alcalin.

9. Procédé selon l'une quelconque des revendications 1 à 8, **caractérisé en ce que** le catalyseur en lit fixe contient du dioxyde de silicium et/ou de l'oxyde d'aluminium comme matériau support.

10. Procédé selon l'une quelconque des revendications 1 à 9, **caractérisé en ce que** la concentration en aldéhyde dans l'alimentation du réacteur est de 1 à 35%.

11. Procédé selon l'une quelconque des revendications 1 à 10, **caractérisé en ce que** la concentration en aldéhyde dans l'alimentation du réaction est de 5 à 20%.

12. Procédé selon l'une quelconque des revendications 1 à 11, **caractérisé en ce qu'**on utilise des mélanges d'hydroformylation préparés à partir d'oléfines en C₈ ou de mélanges d'oléfines en C₈.

13. Procédé selon l'une quelconque des revendications 1 à 11, **caractérisé en ce qu'**on utilise des mélanges d'hydroformylation préparés à partir d'oléfines en C₁₂ ou de mélanges d'oléfines en C₁₂.
